# EUROPEAN PATENT SPECIFICATION

(11) **EP 2 968 188 B1**
(45) Date of publication and mention of the grant of the patent: **19.04.2017**
(21) Application number: 14710734.6
(22) Date of filing: 28.02.2014
(51) Int. Cl.: A61K 9/50, A61K 31/137

(54) **COATED PHENYLEPHRINE PARTICLES AND USE THEREOF IN PHARMACEUTICAL FORMULATIONS**
BESCHICHTETE PHENYLEPHRINPARTIKEL UND VERWENDUNG DAVON IN PHARMAZEUTISCHEN FORMULIERUNGEN
PARTICULES DE PHÉNYLÉPHRINE ENROBÉES ET LEUR UTILISATION DANS DES FORMULATIONS PHARMACEUTIQUES

(30) Priority: 15.03.2013 US 201313832797
(43) Date of publication of application: 20.01.2016
(73) Proprietor: Johnson & Johnson Consumer Inc., Skillman, NJ 08558 (US)
(72) Inventor: LEE, Der-Yang, Flemington, New Jersey 08822 (US); CHEN, Vincent, Upper Holland, Pennsylvania 19053 (US); SHEN, Robert, North Wales, Pennsylvania 19454 (US)
(74) Representative: Carpmaels & Ransford LLP
(86) International application number: PCT/US2014/019311
(87) International publication number: WO 2014/149529

(56) References cited:
- WO-A1-2009/158368
- WO-A2-2008/157103

## Description

### FIELD OF THE INVENTION

The present invention relates to phenylephrine particles suitable for solid, semi solid or liquid dosage forms. The phenylephrine particles, which may be coated, release phenylephrine at rates that provide pharmaceutically suitable plasma concentrations for an extended period of time. The present invention also relates to a process for manufacturing dosage forms containing the phenylephrine particles and to methods for alleviating nasal and respiratory congestion in human subjects with the oral administration of the dosage forms. The dosage forms can further comprise one or more additional therapeutically active agents selected from one or more of the group consisting of antihistamines, decongestants, analgesics, anti-inflammatories, anti-pyretics, cough suppressants and expectorants.

### BACKGROUND OF THE INVENTION

Phenylephrine is a potent vasoconstrictor, possessing both direct and indirect sympathomimetic effects [Hoffman 2001]. The dominant and direct effect is agonism at α1-adrenergic receptors. Stimulation of α1-adrenergic receptors located on capacitance blood vessels of the nasal mucosa (postcapillary venules) results in vasoconstriction, decreased blood volume, and a decrease in the volume of the nasal mucosa (nasal decongestion) [Johnson 1993]. Constricted blood vessels allow less fluid to enter the nose, throat, and sinus linings, which results in decreased inflammation of nasal membranes as well as decreased mucous production [Johnson 1993]. Thus, by constriction of blood vessels, mainly those located in the nasal passages, phenylephrine causes a decrease in nasal congestion [Hoffman 2001, Empey 1981].

Phenylephrine is a Category I (Generally Regarded as Safe and Effective (GRASE)) over-the-counter (OTC) oral nasal decongestant. Globally, phenylephrine has been available since the 1960's, and since 1996, phenylephrine has been widely used in the United States. Phenylephrine hydrochloride, which is widely used in OTC adult and pediatric cough and cold medicines, is indicated for use by adults and children for the temporary relief of nasal congestion due to the common cold, hay fever, or other upper respiratory allergies (allergic rhinitis). It is commercially available in 10 mg tablets for oral administration in adults. The dosing regimen is one 10 mg dose of phenylephrine every four hours, not to exceed 60 mg (six doses) in 24 hours. Complete information is available in the OTC monograph labeling for approved drugs.

Phenylephrine, chemical name (R)-1-(3-hydroxyphenyl)-2-methylaminoethanol, is commercially available as a hydrochloride salt. The empirical formula is C9H13NO2•HCl and the molecular weight is 203.67. The compound, which is a white to off-white crystalline powder, has the following chemical structure:

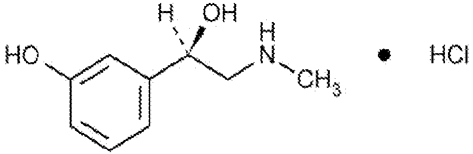

The principal routes of phenylephrine metabolism are sulfate conjugation (mainly in the intestinal wall) and oxidative deamination by both the A and B forms of monoamine oxidase [Suzuki 1979]. Glucuronidation also occurs, but to a lesser extent. In one study, following a 30 mg dose administered orally over eight hours [Ibrahim 1983], phenylephrine was metabolized to phenylephrine-sulfate, m-hydroxymandelic acid, phenylephrine-glucuronide and m-hydroxy-phenylglycol-sulfate at 47%, 30%, 12%, and 6% of the dose, respectively. Deamination is the predominant metabolic pathway after intravenous injection of phenylephrine [Hengstmann 1982], whereas sulfate conjugation is the predominant pathway after oral administration. Phase I and Phase II metabolites of phenylephrine in humans are shown below. The percentage values in the schematic refer to the percent of an oral dose as reported by Ibrahim.

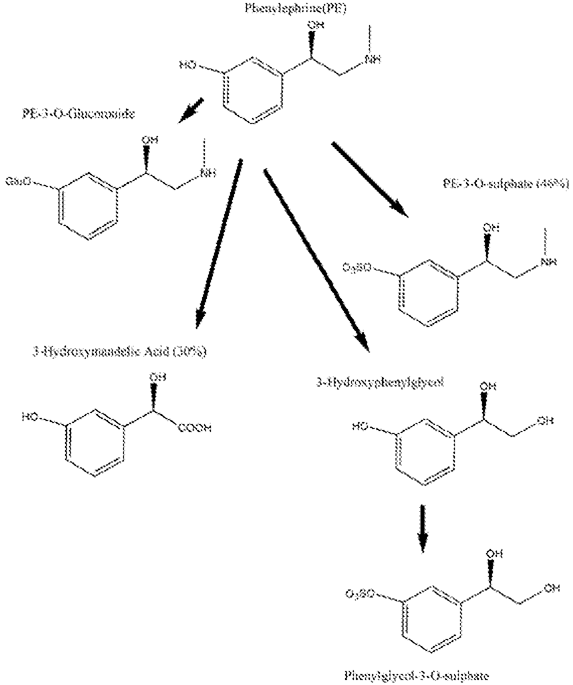

Efficacy data from clinical trials of immediate-release phenylephrine use in adults indicate that phenylephrine is an effective nasal decongestant.

Acetaminophen is a para-aminophenol derivative with analgesic and antipyretic activity. It is used for the temporary relief of minor aches and pains associated with the common cold, backache, headache, toothache, menstrual cramps, and muscular aches; and for the temporary relief of the minor pain of arthritis and for the reduction of fever. The adult dose of acetaminophen in the United States is 1000 mg every four to six hours with a maximum of 4000 mg in 24 hours. The adult dose of extended release acetaminophen is 1300 mg every eight hours with a maximum of 3900 mg in 24 hours.

Acetaminophen is primarily metabolized by the liver via three major parallel pathways: glucuronidation, sulfation, and oxidation [Miners 1983; Slattery 1989; Lee 1992; Miners 1992]. Both the glucuronic and oxidative pathways adhere to a first-order rate process, which means the concentration of acetaminophen metabolized increases as the concentration in the liver increases. The sulfate pathway adheres to Michaelis-Menten kinetics, which means the concentration of acetaminophen metabolized remains constant once the concentration in the liver increases above a saturation level.

A schematic of acetaminophen metabolism is shown below. Less than 9% of a therapeutic dose is excreted unchanged in the urine [Miners 1992]. The major metabolic pathway is glucuronidation, where 47% to 62% of the acetaminophen dose conjugates with glucuronide. These glucuronide conjugates are inactive and nontoxic [Koch-Weser 1976], and are secreted in bile and eliminated in the urine. Glucuronide conjugation is catalyzed primarily by one isoform of glucuronyltransferase (UGT1A6) [Court 2001] with uridine 5'-diphosphoglucuronic acid as an essential cofactor.

The second major pathway of acetaminophen metabolism is sulfation, where 25% to 36% of the dose conjugates with sulfate. These sulfate ester conjugates are also inactive and nontoxic [Koch-Weser 1976], and are readily excreted in the urine. Sulfation is mediated by sulfotransferases, which are heterogeneous cytosolic enzymes, and 3'-phosphoadenosine 5'-phosphate is a cofactor. Sulfotransferase activity rather than sulfate depletion is the rate-controlling factor of acetaminophen sulfation [Blackledge 1991].

The third pathway is oxidation, where 5% to 8% of the acetaminophen dose is metabolized via the cytochrome P-450 enzyme system. The cytochrome P-450 isoenzyme that is primarily responsible for acetaminophen metabolism is CYP2E1 [Manyike 2000]. When acetaminophen is metabolized by CYP2E1, it forms a highly reactive intermediate, N-acetyl-p-benzoquinoneimine (NAPQI). Because NAPQI is highly reactive, it cannot be measured outside the liver nor can it accumulate. This intermediate is rapidly inactivated by hepatocellular stores of glutathione to form cysteine and mercapturate conjugates, which are both inactive and nontoxic [Koch-Weser 1976]. These conjugates are excreted in the urine [Mitchell 1974].

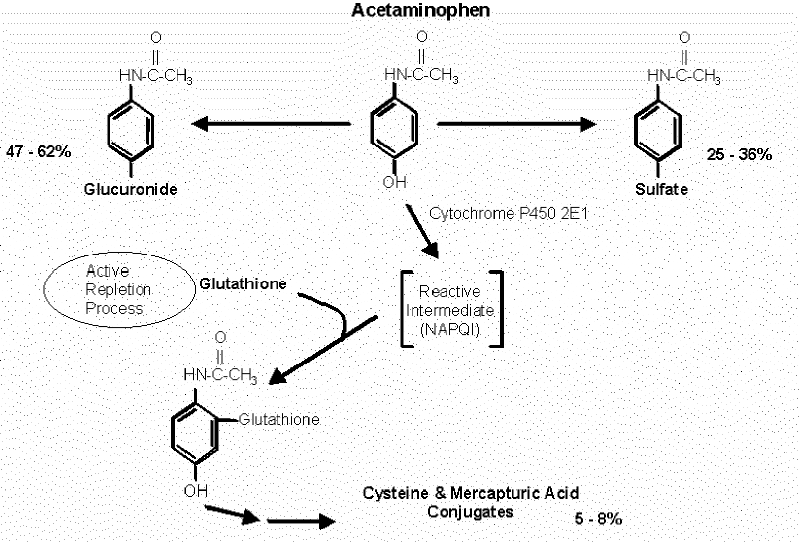

There is a need for less frequent delivery of phenylephrine. Less frequent administration results in improved patient compliance. In addition, constant therapeutic plasma levels of active components can be more effective and even efficacious compared to the fluctuations seen when multiple doses of a conventional immediate release formulation are given. Sustained effective levels could decrease the severity and frequency of side effects seen with high peak plasma levels. Thus, formulations of phenylephrine that can be administered less frequently, for example, once every 6, 8, 12, 16, 20, or 24 hours, are needed.

There is also a need to match the duration of phenylephrine with actives that provide a longer duration than immediate release phenylephrine.

U.S. Published Application No. 20070281020 to Schering-Plough Corporation discloses the administration of a sustained release tablet comprising 30 mg phenylephrine, hydroxypropyl methylcellulose, carboxymethyl cellulose sodium, Kollidon CL-M, colloidal silicon dioxide and magnesium stearate to a human subject and the comparison of the sustained release tablet to three doses of 10 mg immediate release phenylephrine.

U.S. Patent No. 8,282,957 (WO 2009/158368 A1) to McNeil-PPC, Inc. discloses coated phenylephrine particles containing phenylephrine HCl, modified starch and Eudragit NE30D™ coated with a first
coating layer comprising Eudragit RS PO, acetyltributylcitrate and magnesium stearate and a second coating layer comprising Eudragit NE30D™, Eudragit FS30D™, magnesium stearate, sodium lauryl sulfate and simethicone, and use thereof in pharmaceutical dosage forms, including dosage forms containing acetaminophen.

U.S. Patent No. 6,001,392 to Warner Lambert Company discloses a drug/resin complex that contains a mixture of coated and uncoated Amberlite™ IR69 cross-linked with divinylbenzene.

U.S. Published Application No. 20100068280 to Schering-Plough Corporation discloses pharmaceutical dosage forms comprising phenylephrine in sustained release form. According to an embodiment, a single dose of phenylephrine in a tablet containing 30 mg phenylephrine, lactose monohydrate, Methocel K100M CR, Klucel EXF and magnesium stearate was compared to two 10 mg phenylephrine immediate release tablets dosed 4 hours apart in a bioequivalence study.

U.S. Published Applications Nos. 20050266032 and 20060057205 to Sovereign Pharmaceuticals disclose pharmaceutical dosage forms containing phenylephrine. According to an embodiment, the phenylephrine is incorporated into an ion-exchange resin complex using, e.g., sodium polystyrene sulfonate, and coated with delayed release polymer, e.g., Eudragit® L 100, Kollidon® MAE and Aquacoat® cPD. The formula in this embodiment contains 45 mg sustained release phenylephrine and 15 mg immediate release phenylephrine.

U.S. Patent No. 8,062,667 to Tris Pharma, Inc. discloses coated drug-ion exchange resin complexes. According to an embodiment, phenylephrine is incorporated into an ion-exchange resin complex using, sodium polystyrene sulfonate, and coated with KOLLICOAT™ SR-30D, triacetin and water.

U.S. Patent No. 8,394,415 to McNeil-PPC, Inc. discloses a liquid formulation comprising immediate release ibuprofen and an extended release phenylephrine-specified ion exchange resin complex coated with first and second coating layers comprising specified ingredients.

U.S. Application No. 11/761,698 (WO 2008/157103 A2) to McNeil-PPC, Inc. discloses a solid composition comprising ibuprofen (IR) and phenylephrine coated with first coating layer comprising ethylcellulose and second coating layer comprising protective coating.

U.S. Application No. 20100068280 to Schering-Plough Healthcare Products, Inc. discloses a bioavailability study that compared 10 mg phenylephrine HCl delivered via Enterion™ capsules, 10 mg Sudafed PE™ and 30 mg phenylephrine HCl delivered via Enterion™ capsules.

U.S. Patent Application No. 2007014239 to Coating Place, Inc. discloses a method and composition for loading one or more drugs onto one or more ion exchange resin particles to form a drug loaded resin particle.

There continues to be a need for phenylephrine products having the attributes discussed above.

### SUMMARY OF THE INVENTION

The present invention is directed to phenylephrine particles that deliver phenylephrine or a pharmaceutically acceptable salt thereof to a subject in need thereof so as to provide a peak plasma concentration of phenylephrine at about 0.1 to about 16 hours, preferably about 0.5 to about 5 hours, more preferably about 1 to about 4.5 hours, after ingestion and wherein the phenylephrine is maintained at a level greater than about 20, about 40, about 60, about 80, about 100, about 120, about 140, about 160, about 180, or about 200, pg/mL for at least about 6, about 8, about 12, about 16, about 20 and/or about 24 hours after ingestion.

Also described herein are coated phenylephrine resinate particles that deliver phenylephrine or a pharmaceutically acceptable salt thereof to a subject in need thereof so as to provide a peak plasma concentration of phenylephrine at about 0.1 to about 16 hours, preferably about 0.5 to about 5 hours, more preferably about 1 to about 4.5 hours, after ingestion and wherein the phenylephrine is maintained at a level greater than about 20, about 40, about 60, about 80, about 100, about 120, about 140, about 160, about 180, or about 200, pg/mL for at least about 6, about 8, about 12, about 16, about 20 and/or about 24 hours after ingestion.

The present invention is also directed to pharmaceutical dosage forms comprising phenylephrine particles that deliver phenylephrine or a pharmaceutically acceptable salt thereof to a subject in need thereof so as to provide a peak plasma concentration of phenylephrine at about 0.1 to about 16 hours, preferably about 0.5 to about 5 hours, more preferably about 1 to about 4.5 hours, after ingestion and wherein the phenylephrine is maintained at a level greater than about 20, about 40, about 60, about 80, about 100, about 120, about 140, about 160, about 180 or about 200, pg/mL for at least about 6, about 8, about 12, about 16, about 20 and/or about 24 hours after ingestion.

In another embodiment, the phenylephrine particles, which provide extended release of phenylephrine, are combined with phenylephrine in immediate release form.

In another embodiment, the phenylephrine particles are combined with one or more additional therapeutic agent(s) for immediate or sustained release. Such agent or agents may be formulated for immediate release upon ingestion, for sustained release, for release in the colon concomitantly with at least some of the phenylephrine, or any combination thereof. In one embodiment, the additional therapeutic agent is uncoated. In another embodiment, the additional therapeutic agent is coated.

The additional therapeutic agent may be an antihistamine, a decongestant, an analgesic, an anti-inflammatory, an anti-pyretic, a cough suppressant, an expectorant, or any other therapeutic agent or combinations of such agents useful to alleviate the symptoms of a cold, seasonal and other allergies, hay fever, or sinus problems, any of which may cause an increase in nasal discharge. Preferably, the one or more additional therapeutic agents are acetaminophen.

Examples of antihistamines and decongestants, include, but are not limited to, bromopheniramine, chlorcyclizine, dexbrompheniramine, bromhexane, phenindamine, pheniramine, pyrilamine, thonzylamine, pripolidine, ephedrine, pseudoephedrine, phenylpropanolamine, chlorpheniramine, dextromethorphan, diphenhydramine, doxylamine, astemizole, terfenadine, fexofenadine, naphazoline, oxymetazoline, montelukast, propylhexadrine, triprolidine, clemastine, acrivastine, promethazine, oxomemazine, mequitazine, buclizine, bromhexine, ketotifen, terfenadine, ebastine, oxatamide, xylomeazoline, loratadine, desloratadine, and cetirizine; isomers thereof, and pharmaceutically acceptable salts and esters thereof.

Examples of suitable analgesics, anti-inflammatories, and antipyretics include, but are not limited to, non-steroidal anti-inflammatory drugs (NSAIDs) such as propionic acid derivatives (e.g., ibuprofen, naproxen, ketoprofen, flurbiprofen, fenbufen, fenoprofen, indoprofen, ketoprofen, fluprofen, pirprofen, carprofen, oxaprozin, pranoprofen, and suprofen) and COX inhibitors such as celecoxib; acetaminophen; acetyl salicylic acid; acetic acid derivatives such as indomethacin, diclofenac, sulindac, and tolmetin; fenamic acid derivatives such as mefanamic acid, meclofenamic acid, and flufenamic acid; biphenylcarbodylic acid derivatives such as diflunisal and flufenisal; and oxicams such as piroxicam, sudoxicam, isoxicam, and meloxicam; isomers thereof, and pharmaceutically acceptable salts and prodrugs thereof.

Examples of cough suppressants and expectorants include, but are not limited to, diphenhydramine, dextromethorphan, noscapine, clophedianol, menthol, benzonatate, ethylmorphone, codeine, acetylcysteine, carbocisteine, ambroxol, belladona alkaloids, sobrenol, guaiacol, and guaifenesin; isomers thereof, and pharmaceutically acceptable salts and prodrugs thereof.

Another aspect of the invention is a method of treating the symptoms of cold, influenza, allergies, or non-allergic rhinitis in a subject in need thereof comprising administering the phenylephrine particles of the invention. In certain embodiments, the phenylephrine particles are administered about every 6, 8, 12, 16, 20, or 24 hours. In one preferred embodiment, the phenylephrine particles are administered about every 12 hours. In another preferred embodiment, the phenylephrine particles are administered about every 8 hours.

Certain embodiments of the invention are methods of maintaining sustained bioavailability of phenylephrine in a subject, comprising orally administering to the subject phenylephrine particles, wherein at least a portion of phenylephrine is absorbed from the colon, and wherein the concentration of phenylephrine in the plasma of the subject is at least about 20, about 40, about 60, about 80, about 100, about 120, about 140, about 160, about 180, or about 200, pg/mL at about 6 hours after administration of the composition. In particular embodiments, the concentration of phenylephrine in the plasma of the subject is at least about 20, about 40, about 60, about 80, about 100, about 120, about 140, about 160, about 180, or about 200, pg/mL at about 8 hours after administration of the composition. In particular embodiments, the concentration of phenylephrine in the plasma of the subject is at least about 20, about 40, about 60, about 80, about 100, about 120, about 140, about 160, about 180, or about 200, pg/mL at about 12 hours after administration of the composition. In particular embodiments, the concentration of phenylephrine in the plasma of the subject is at least about 20, about 40, about 60, about 80, about 100, about 120, about 140, about 160, about 180, or about 200, pg/mL at about 20 hours after administration of the composition. In particular embodiments, the concentration of phenylephrine in the plasma of the subject is at least about 20, about 40, about 60, about 80, about 100, about 120, about 140, about 160, about 180, or about 200, pg/mL at about 24 hours after administration of the composition. Certain other embodiments of the invention are methods of administering phenylephrine to a subject, comprising orally administering phenylephrine particles, said composition delivering at least some of the phenylephrine to the colon where phenylephrine is released in the colon and absorbed from the colon.

The present invention may be more fully understood by reference to the Figures, Detailed Description and Examples which follow.

### BRIEF DESCRIPTION OF THE FIGURES

Figures relating to phenylephrine resinate particles are for reference only.

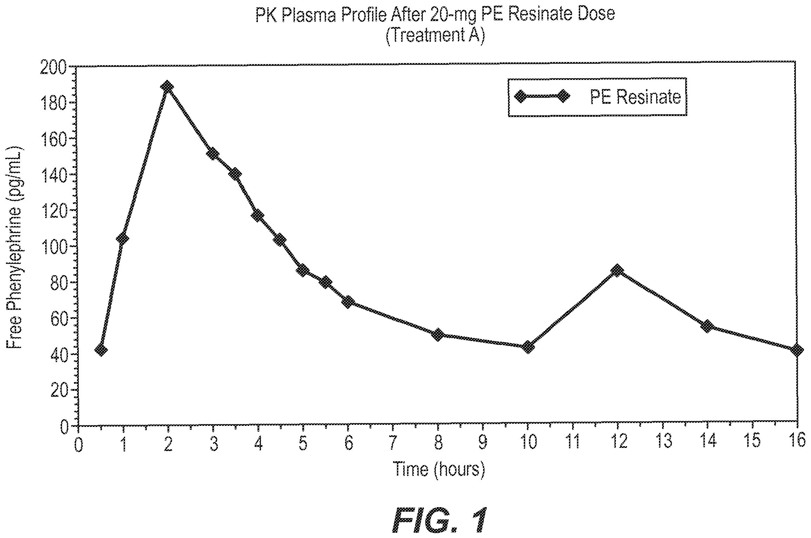

Figure 1 shows the mean plasma concentration profile of phenylephrine upon administration of coated extended release (ER) phenylephrine resinate particles containing 20 mg phenylephrine. Referring to Figure 1, the y axis represents the concentration of free phenylephrine in plasma in picograms (pg) per milliliter (mL). The x axis represents time in hours. Figure 1 shows that the average concentration of phenylephrine reached a peak (Cmax) at about 2 hours. Figure 1 also shows a secondary peak at about 12 hours.

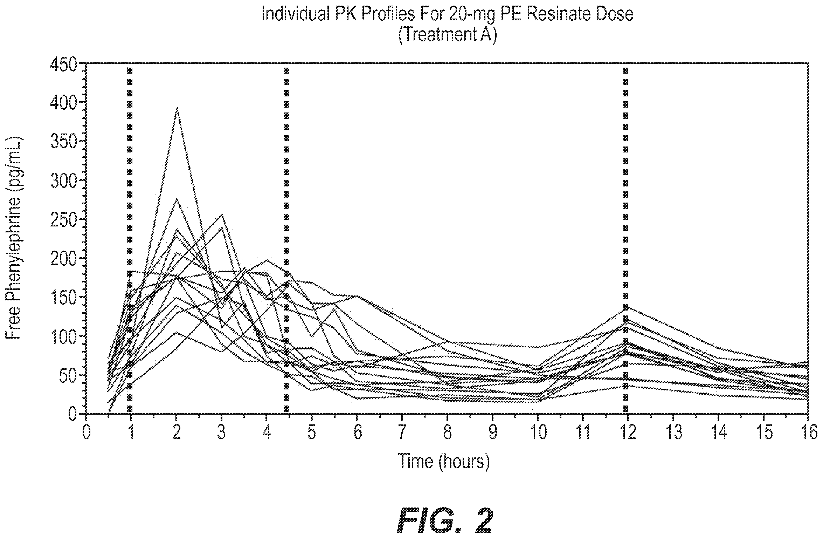

Figure 2 shows the individual plasma concentration profiles of phenylephrine upon administration of coated ER phenylephrine resinate particles containing 20 mg phenylephrine. Referring to Figure 2, the intersubject variability is good for modified release phenylephrine. The range of Cmax occurred from about 1 hour to about 4.5 hours. The secondary peak at about 12 hours was observed for all subjects.

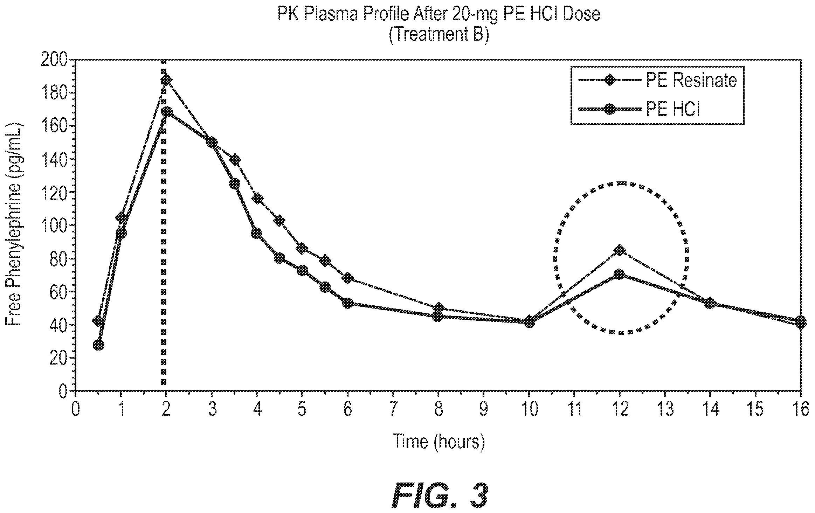

Figure 3 shows the mean plasma concentration profile of phenylephrine upon administration of coated ER phenylephrine HCl particles containing 20 mg phenylephrine. Referring to Figure 3, the dashed line is the profile from Figure 1 for comparison purposes. A slightly higher Cmax with the phenylephrine resinate particles was observed. A secondary peak at about 12 hours is observed in both profiles. This may be the result of less phenylephrine being metabolized presystemically by the gut wall as a result of the particles quick movement down the GI tract. Release of phenylephrine in the colon would result in higher absorption at a later time.

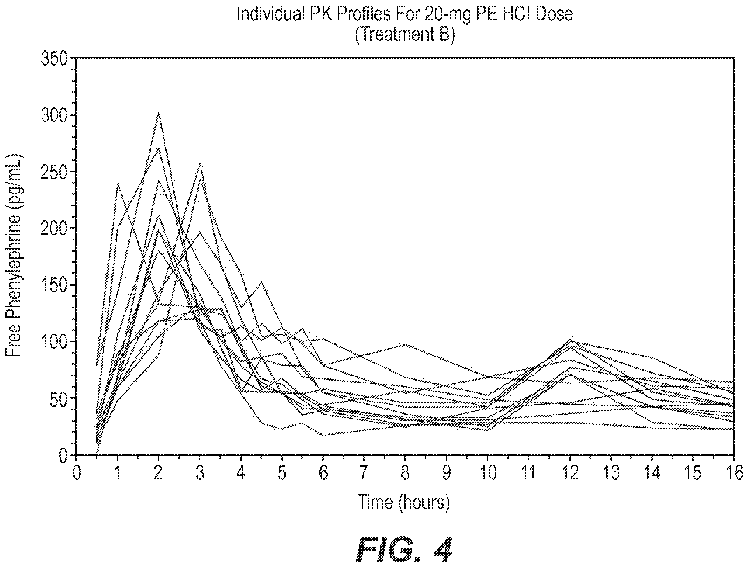

Figure 4 shows the individual plasma concentration profiles of phenylephrine upon administration of coated ER phenylephrine HCl particles containing 20 mg phenylephrine.

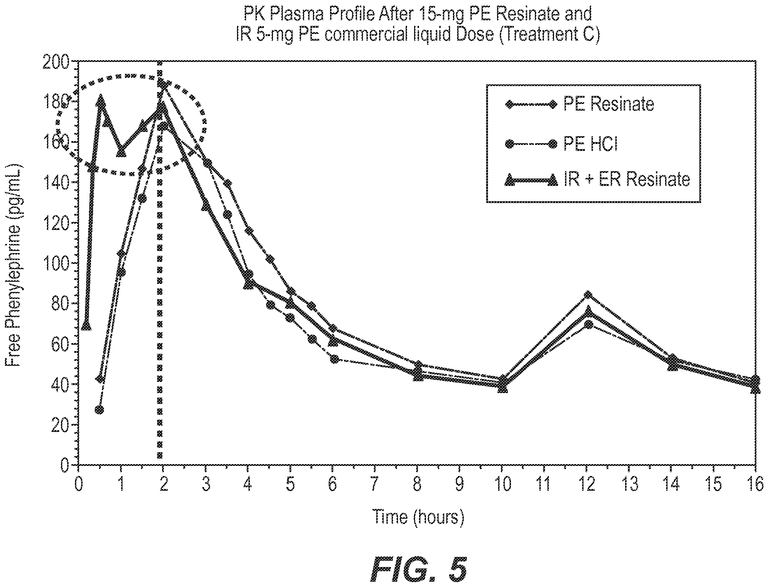

Figure 5 shows the mean plasma concentration profile of phenylephrine upon administration of coated ER phenylephrine resinate particles containing 15 mg phenylephrine and liquid IR phenylephrine HCl containing 5 mg phenylephrine (the "ER-IR blend"). Referring to Figure 5, the unbroken line represents the ER-IR blend. Again, the curve for this treatment is consistent with what was seen with the resinate and the HCl formulations. For the ER-IR blend, there are two peaks within the first 2 hours; one mainly from the IR dose and the other from the accumulation of the IR and ER doses. The Cmax was reached faster and maintained for a longer period of time. An ER-IR blend thus appears beneficial in terms of onset of efficacy.

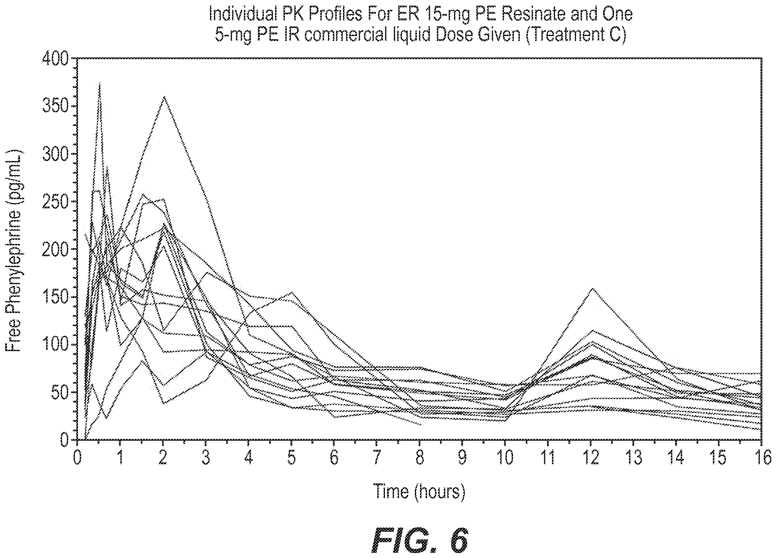

Figure 6 shows the individual plasma concentration profiles of phenylephrine upon administration of coated ER phenylephrine resinate particles containing 15 mg phenylephrine and liquid IR phenylephrine HCl containing 5 mg phenylephrine.

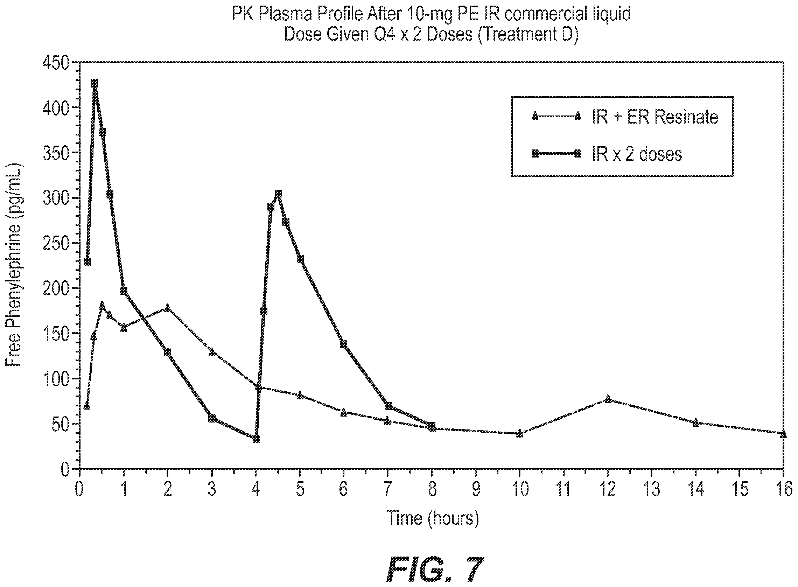

Figure 7 shows the mean plasma concentration profile of phenylephrine upon administration of liquid IR phenylephrine HCl containing 20 mg phenylephrine. Referring to Figure 7, the unbroken line represents the profile for the currently marketed IR liquid product and the dashed line is the profile from Figure 5 for comparison. The Cmax of the ER-IR blend is lower than the Cmax of the IR form.

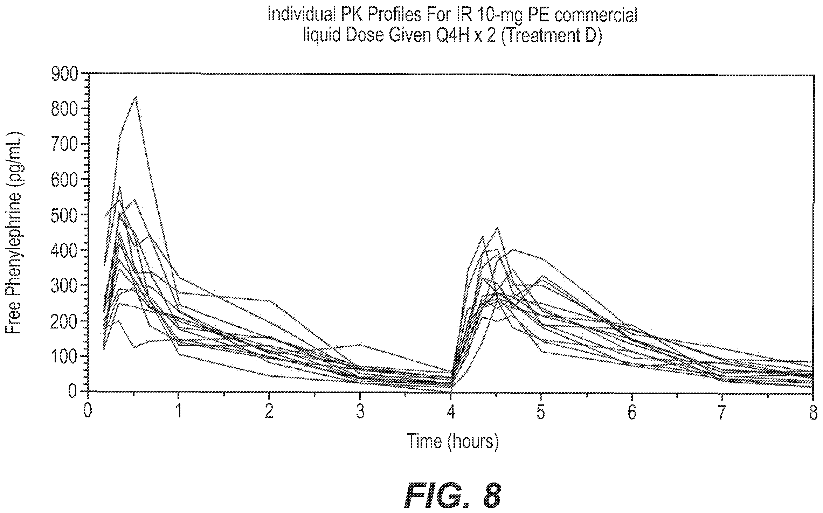

Figure 8 shows the individual plasma concentration profiles of phenylephrine upon administration of liquid IR phenylephrine HCl containing 20 mg phenylephrine.

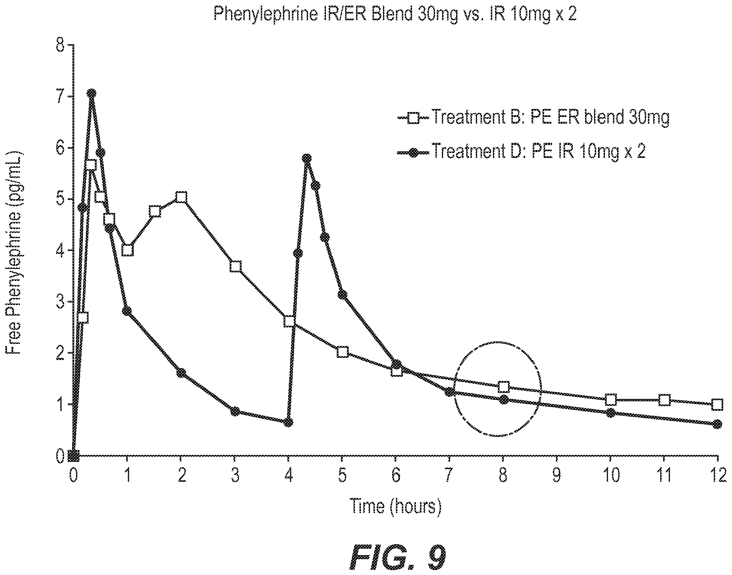

Figure 9 shows the mean plasma concentration profile of phenylephrine upon administration of coated ER phenylephrine resinate particles containing 22.5 mg phenylephrine and liquid IR phenylephrine HCl containing 7.5 mg phenylephrine (the "ER-IR blend") and compares to liquid IR phenylephrine HCl containing 20 mg phenylephrine.

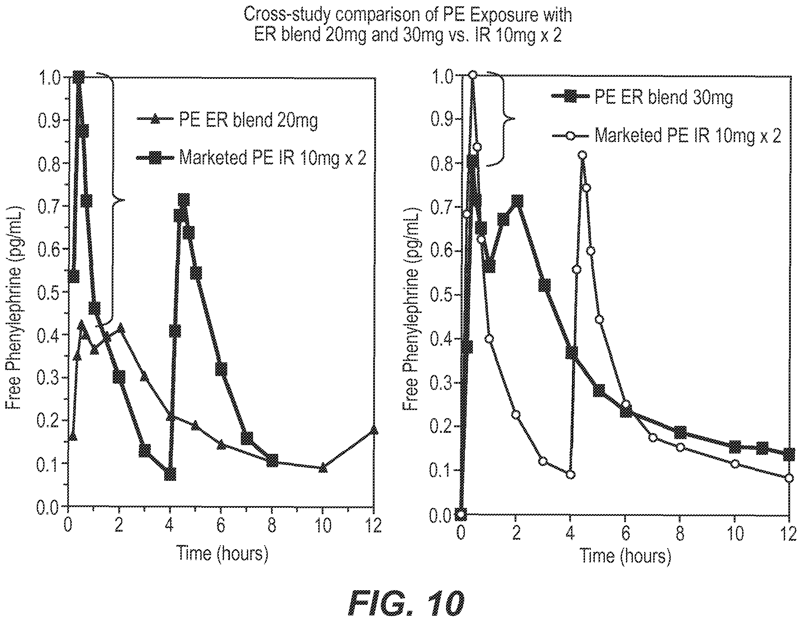

Figures 10A and 10B compare the mean plasma concentration profile of phenylephrine (1) upon administration of coated ER phenylephrine resinate particles containing 15 mg phenylephrine and liquid IR phenylephrine HCl containing 5 mg phenylephrine (Figure 10A) and (2) upon administration of coated ER phenylephrine resinate particles containing 22.5 mg phenylephrine and liquid IR phenylephrine HCl containing 7.5 mg phenylephrine (Figure 10B) with (3) liquid IR phenylephrine HCl containing 20 mg phenylephrine.

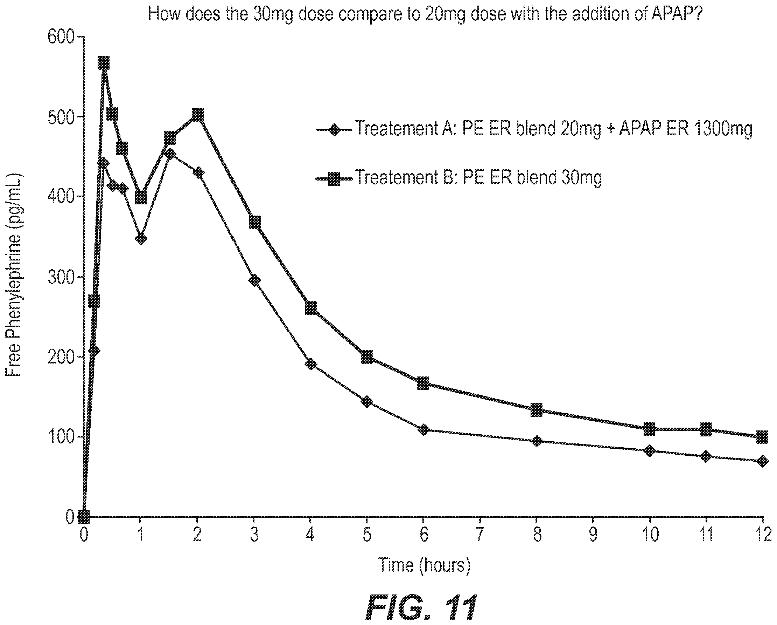

Figure 11 compares the mean plasma concentration profiles of phenylephrine upon administration of (1) coated ER phenylephrine resinate particles containing 15 mg phenylephrine and liquid IR phenylephrine HCl containing 5 mg phenylephrine with a (2) combination of (a) coated ER phenylephrine resinate particles containing 15 mg phenylephrine, (b) liquid IR phenylephrine HCl containing 5 mg phenylephrine and (c) 1300 mg ER acetaminophen.

### DETAILED DESCRIPTION OF THE INVENTION

It is believed that one skilled in the art can, based upon the description herein, utilize the present invention to its fullest extent. The following specific embodiments are to be construed as merely illustrative, and not as limiting the remainder of the disclosure in any way whatsoever.

Unless defined otherwise, all technical and scientific terms used herein have the same meaning as commonly understood by one of ordinary skill in the art to which the invention belongs. As used herein, all percentages are by weight unless otherwise specified. In addition, all ranges set forth herein are meant to include any combinations of values between the two endpoints, inclusively.

### DEFINITIONS

As used herein a pharmaceutically acceptable salt of phenylephrine includes, but is not limited to, phenylephrine hydrochloride, phenylephrine bitartrate, phenylephrine tannate, etc. In one preferred embodiment, the pharmaceutically acceptable salt of phenylephrine is phenylephrine hydrochloride.

"AUC" as used herein means, for any given drug, the "area under the concentration-time curve" from dosing or activation of the drug to a time point, calculated by the trapezoidal rule. AUC is a parameter showing the cumulative plasma concentration of a drug over time, and is an indicator of the total amount and availability of a drug in the plasma.

"Cmax" as used herein means the maximum (or peak) concentration that a drug achieves in tested area after the drug has been administrated and prior to the administration of a second dose.

As used herein, "crystalline form" shall mean the non-amorphous form of the active ingredient such that it displays crystal like properties including, but not limited to, the ability to diffract visible light. Crystalline may also be used to describe an active ingredient in its pure form, i.e., e.g., without the addition of other excipients thereto.

By "delayed release," it is meant that, after administration, there is at least one period of time when an active ingredient is not being released from the dosage form, i.e., the release of the active ingredient(s) occurs at a time other than immediately following oral administration.

As used herein, "dissolution medium" shall mean any suitable liquid environment in which the suspension dosage form of the present invention can be dissolved, such as, for example, the in vitro dissolution media used for testing of the product, or gastro-intestinal fluids. Suitable in vitro dissolution media used for testing the dissolution of the active ingredient or ingredients from the suspension dosage form of the present invention include those described in the United States Pharmacopeia.

A "dosage", "dosage form" or "dose" as used herein means the amount of a pharmaceutical composition comprising therapeutically active agent(s) administered at a time. "Dosage", "dosage form" or "dose" includes administration of one or more units of pharmaceutical composition administered at the same time. In one embodiment, the dosage form is a tablet. In one embodiment the dosage form is a multilayer tablet. In the embodiment comprising a multilayer tablet, one layer may comprise an immediate release portion and another layer may comprise an extended release portion. In an aspect of the disclosure comprising a multilayer tablet, one layer may comprise the phenylephrine resinate particles, and another layer may comprise an immediate release form of phenylephrine and/or a second active ingredient. In one aspect of the disclosure, the dosage form comprising phenylephrine resinate particles is a liquid filled soft-gel.

As described herein "drug-resin complex" shall mean the bound form of an active ingredient, including but not limited to the pharmaceutical active ingredients, and an ion exchange resin. The drug-resin complex is also referred to in the art as a "resinate." An ion exchange resin described herein is Amberlite™ IRP 69, The Dow Chemical Company, an insoluble, strongly acidic, sodium form cationic exchange resin derived from sulfonated copolymer of styrene and divinylbenzene. The mobile, or exchangeable cation is sodium, which can be exchanged for, or replaced by, many cationic (basic) species, including, e.g., copper, zinc, iron, calcium, strontium, magnesium and lithium. Adsorption of drug onto ion exchange resin particles to form the drug/resin complex is a well known technique as shown in U.S. Patents Nos. 2,990,332 and 4,221,778. In general the drug is mixed with an aqueous suspension of the resin, and the complex is then washed and dried. Adsorption of drug onto the resin may be detected by measuring a change in the pH of the reaction medium, or by measuring a change in concentration of sodium or drug. The drug/resin complex formed can be collected and washed with ethanol and/or water to insure removal of any unbound drug. The complexes are usually air-dried in trays at room or elevated temperature. The drug/resin complex has a ratio of phenylephrine to resin of about 0.25:1 to about 0.65:1, preferably about 0.30:1 to about 0.55:1, preferably about 0.35:1 to about 0.45:1.

"Enteric" shall mean being able to be dissolved at a pH of greater than about 5.0 or greater than about 5.5 or greater than about 6.0 or that which is found in the intestine.

By "extended release," it is meant that, after administration, an active ingredient is released from the dosage form in a substantially continuous, regulated manner, and the time for complete release, i.e., depletion, of the active ingredient from the dosage form is longer than that associated with an immediate release dosage form of the same. Types of extended release include controlled, sustained, prolonged, zero-order, first-order, pulsatile, and the like.

As used herein, "immediate release" means that the dissolution characteristics of at least one active ingredient meet USP specifications for immediate release tablets containing that active ingredient. An active ingredient having an immediate release property may be dissolved in the gastrointestinal contents, with no intention of delaying or prolonging the dissolution of the active ingredient.

"Liquid dosage forms" may nonexclusively include suspensions or elixirs, wherein one or more of the active ingredients is dissolved, partially dissolved or in an undissolved or suspended state.

As used herein, "modified release" shall apply to the altered release or dissolution of an active ingredient in a dissolution medium, such as gastrointestinal fluids. Types of modified release include: 1) extended release; or 2) delayed release. In general, modified release dosage forms are formulated to make the active ingredient(s) available over an extended period of time after ingestion, which thereby allows for a reduction in dosing frequency compared to the dosing of the same active ingredient(s) in a conventional dosage form. Modified release dosage forms also permit the use of active ingredient combinations wherein the duration of one active ingredient may differ from the duration of another active ingredient.

As used herein, "pharmacodynamics" or "PD" is the study of the relationship between drug concentration at the site of action and the resulting effect.

As used herein, "pharmacokinetics" or "PK" is the study of the time course of drug absorption, distribution, metabolism and excretion.

As used herein, the term "phenylephrine" means benzynemethanol, 3-hydroxy-α-[(methylamino)methyl], and includes, but is not limited to pharmaceutically acceptable salts, esters, isomers or derivatives thereof.

As used herein, a drug "release rate" refers to the quantity of drug released from a dosage form per unit time, e.g., milligrams of drug released per hour (mg/hr). Drug release rates are calculated under in vitro dosage form dissolution testing conditions known in the art. As used herein, a drug release rate obtained at a specified time "following administration" refers to the in vitro drug release rate obtained at the specified time following commencement of an appropriate dissolution test, e.g., those set forth in USP 24 (United States Pharmacopeia 24, United States Pharmacopeia Convention, Inc., Rockville, MD).

"Semipermeable," as used herein, shall mean that water can pass through, and other molecules, including salts and the active ingredients described herein, are allowed to slowly diffuse through such a membrane when the membrane is in contact with an appropriate dissolution medium, e.g., gastro-intestinal fluids or in-vitro dissolution media.

"Semi-solid dosage forms" shall mean dosage forms which are highly viscous and share some of the properties of liquids, including but not limited to (1) having the ability to substantially conform to something that applies pressure to it and causes its shape to deform; and (2) lacking the ability to flow as easily as a liquid. Semi-solid dosage forms also share some of the properties of solids, including but not limited to having a higher density and a defined shape. Semi-solids may nonexclusively include gels, chewy dosage forms, pectin based chewy forms, confectionery chewy forms, moldable gelatin type of forms.

"Solid dosage forms" shall mean dosage forms which are substantially solid at room temperature and have a density of at least about 0.5 g/cc. Solid dosage forms may non exclusively include, agglomerated tablets, capsule-like medicaments, powder or granule filled capsules, powder or granule filled sachets, compressed tablets, coated tablets, chewable dosage forms, and fast-dissolving dosage forms.

As used herein, "substantially coated" with regard to particles shall mean that less than about 20%, e.g., less than about 15%, or less than about 1.0% of the surface area of the particle is exposed, e.g., not covered, with a desired coating. As used herein, the term "substantially covers" or "substantially continuous" when used to describe a coating means that the coating is generally continuous and generally covers the entire surface of the core or underlying layer, so that little to none of the active ingredient or underlying layer is exposed. The coatings which are applied to the particles can be layered wherein each layer is prepared in an aqueous (water based) or organic solvent system and added in succession until the desired coating level is achieved.

"Therapeutic effect," as used herein, shall mean any effect or action of an active ingredient intended to diagnose, treat, cure, mitigate, or prevent disease, or affect the structure or any function of the body.

Specific embodiments of the present invention are illustrated by way of the following examples.

This invention is not confined to the specific limitations set forth in these examples.

### EXAMPLES

Any examples herein relating to resinate particles are included for reference only.

Phenylephrine extended release particles were developed in order to formulate into liquid and solid dosage forms. The phenylephrine extended release particles can be used to match duration with other actives (particularly pain actives) which may provide a longer duration than phenylephrine. Such actives include, but are not limited to, acetaminophen, ibuprofen and naproxen and salts and derivatives thereof.

### Example 1: Preparation of Formulation Containing Coated Phenylephrine Extended Release Particles

A formulation that contains phenylephrine particles coated with a polymer coating was prepared. The formulation, which provides release of phenylephrine over an extended period of time, has proven to be stable at 25°C/60%RH for 24 months and at 40°C/75%RH for 3 months. Many granulated formulations of phenylephrine are not stable over time and undergo significant degradation.

A batch of 3.203 kg of coated phenylephrine particles was prepared according to the formula in Table 1. The quantitative and batch formulas, respectively, are represented in Table 1.

**Table 1: Coated Extended Release Phenylephrine Particles¹**

| Component | Weight/Unit Dose (mg) | Weight % (w/w) | Weight/Batch (kg) |
|---|---|---|---|
| Phenylephrine HCl USP | 20.00 | 5.30 | 0.1699 |
| Pregelatinized Modified Starch NF | 91.22 | 24.17 | 0.7739 |
| Ethyl Acrylate and Methyl Methacrylate Copolymer Dispersion (Eudragit® NE 30D) NF | 134.66 | 35.68² | 1.1427 |
| Ethylcellulose (Ethocel® Standard Premium 10) NF | 45.33 | 12.01 | 0.3846 |
| Acetyltributyl Citrate NF | 9.06 | 2.40 | 0.0769 |
| Magnesium Stearate NF | 9.06 | 2.40 | 0.0769 |
| Ethylcellulose Aqueous Dispersion (Aquacoat ECD®)³ NF | 66.27 | 17.56² | 0.5625 |
| Colloidal Silicon Dioxide NF | 1.77 | 0.47 | 0.0150 |
| Purified Water⁴ USP | --- | --- | --- |
| Acetone⁴ NF | --- | --- | --- |
| Isopropyl Alcohol⁴ NF | --- | --- | --- |
| TOTAL | | 100.00 | 3.203 |

| | | | |
|---|---|---|---|
| 1: A unit dose of the particles containing 20 mg phenylephrine HCl is approximately 377.4 mg. Actual weight is dependent on the assayed amount of phenylephrine HCl in the particles. 2: Solids weight. 3: Contains ethylcellulose, cetyl alcohol and sodium lauryl sulfate. 4: Purified water, acetone and isopropyl alcohol are removed during processing. | | | |

### Layering of Particles:

1. Purified water USP was added to a suitably sized stainless steel container.
2. Ethyl acrylate NF and methyl methacrylate copolymer dispersion NF (Eudragit® NE 30D) was added with gentle agitation.
3. Phenylephrine HCl USP was added while mixing with agitation and mixed.
4. Step 3 mixture was used to coat (layer) pregelatinized modified starch NF.

### Drying and Screening:

5. The layered phenylephrine HCl/pregelatinized modified starch from Step 4 was dried and screened through a #20 screen.

### Coating of Layered Particles with Ethylcellulose Coating Solution:

6. The following were added in the order as they appear to a suitably sized container with gentle agitation: isopropyl alcohol USP, followed by acetone NF, followed by acetyltributyl citrate NF.
7. Ethylcellulose NF (Ethocel® Standard Premium 10) was added with agitation and mixed until a clear solution was formed.
8. Magnesium stearate was added to the solution with agitation.
9. The screened layered phenylephrine/pregelatinized modified starch particles from Step 5 were coated with the solution from Step 8 using a suitable fluid bed coating unit fitted with a Wurster insert.

### Curing:

10. The particles from Step 9 were cured in an oven.

### Coating of Ethocel® Coated Particles with Eudragit® NE30D and Aquacoat ECD®:

11. Eudragit® NE30D was added followed by purified water USP and ethylcellulose aqueous dispersion NF (Aquacoat ECD®) to a suitably sized container and mixed with gentle agitation.
12. The Ethocel® coated layered particles from Step 10 were coated with the coating solution using a suitable fluid bed coating unit fitted with a Wurster insert.
13. The coated particles from Step 12 were mixed with colloidal silicon dioxide NF.

### Curing:

14. The particles from Step 13 were cured in an oven.

### Dissolution Analysis

The coated phenylephrine particles from Step 14 were analyzed for dissolution from 0 to 14 hours using the apparatus described in United States Pharmacopeia <General Chapter <711> Dissolution>, Apparatus II, rotating paddles, utilizing UV detection at 274 nm. The dissolution media was 750 mL of 0.1N HCl for the first hour and then was 1000 mL of a 0.05 M sodium phosphate buffer, pH 6.8, for the second to the fourteenth hour. The temperature was 37°C and the rotation speed was 50 rpm. The dissolution showed that the percent of phenylephrine released versus a standard prepared at 100% of the amount of phenylephrine in the formulation was less than or equal to 50% in 1 hour, greater than or equal to 30% in 3 hours and greater than or equal to 50% in 8 hours. The method employed is below and the results are shown in Table 2 below.

### Dissolution method USP Apparatus (2 paddles, 50 rpm)

1. Verify that the dissolution media temperature has reached the target value (37°C).
2. Weigh out samples equivalent to 45 mg of phenylephrine HCl. Add samples (onto the surface of the medium solution) to each vessel containing 750 mL of 0.1N hydrochloric acid and start the dissolution test with the paddle speed at 50 rpm. After 1 hour of operation in 0. IN hydrochloric acid, complete the 1 hour time point measurement. Proceed immediately to the buffer stage by adding 250 mL of 0.20 M tribasic sodium phosphate. The pH of the buffer medium is 6.8 ± 0.05.
3. Measure the UV absorbance of phenylephrine HCl released in the medium by using a LEAP fiber optic system with in line probes for UV measurement at 274 nm.
4. The amount of phenylephrine HCl dissolved can be determined using the UV absorbance of the sample solution under test in comparison with that of a standard solution at the wavelength of 274 nm. The amount of phenylephrine HCl dissolved can also be determined using the assay method below.

**Table 2**

| Time (hours) | % Dissolution |
|---|---|
| 1 | 10%-30% |
| 2 | 30%-50% |
| 3 | 50%-70% |
| 4 | 60%-80% |
| 6 | 75%-95% |
| 8 | 85%-100% |
| 10 | 90%-100% |
| 12 | 90%-100% |
| 14 | 90%-100% |

### Assay method

### Sample preparation

1. Accurately weigh approximately 1600 mg of phenylephrine HCl particles and transfer into a 200-mL volumetric flask. (It is recommended to add 1 mL of 1% acetic acid / water solution to wet the particles to avoid the formation of solid clumps).
2. Add 70 mL of 1% acetic acid / acetonitrile solution; shake the flask on a platform shaker at low speed for 1 hour. Note: swirl the flask periodically to remove the particles collected above the solvent level.
3. Add about 50 mL of 1% acetic acid / water solution to the flask and continuously shake the flask at low speed for 1 hour.
4. Dilute to volume with 1% acetic acid / water solution and mix well.
5. Filter an aliquot using a 0.45µm Millipore Millex PVDF filter. Discard the first 1-2
   mL of filtrate before collection the filtrate for the further dilution.
6. Pipet 6 mL of the filtrate into a 50-mL volumetric flask, dilute to volume with 1%
   acetic acid / water and mix well.

### Analysis of Phenylephrine

Inject standards (0.05 mg/mL of phenylephrine HCl in 1% acetic acid / water) and samples onto a suitable HPLC system under conditions similar to those suggested below. Parameters may be modified to optimize chromatography. Determine the assay of phenylephrine HCl using the peak areas of the sample solutions under test in comparison with the peak areas of the standard solution.

### HPLC Chromatographic Conditions

| | |
|---|---|
| Column | Phenomenex Luna SCX, 100 mm length x 4.6 mm ID, 5 µm particle size, 100 Angstrom pore size |
| Mobile Phase | 25 mM Sodium Acetate Trihydrate Buffer (pH 4.6):Acetonitrile (65:35, v/v) |
| Mobile Phase Program | Isocratic |
| Detector | UV, 214 nm |
| Flow Rate | 2.0 mL/min |
| Injection Volume | 100 µL |
| Column Temperature | Ambient |
| Suggested Run Time | 7 minutes |
| Approx. Retention Time | PHE 5 min |

### Degradation Products Method

### Sample preparation

2. Accurately weigh approximately 1600 mg of phenylephrine HCl particles and transfer into a 200-mL volumetric flask. (It is recommended to add 1 mL of 1% Acetic Acid / water Solution to wet the particles to avoid the formation of solid clumps).
2. Add 70 mL of 1% acetic acid / acetonitrile solution; shake the flask on a platform shaker at low speed for 1 hour. Note: swirl the flask periodically to remove the particles collected above the solvent level.
3. Add about 50 mL of 1% acetic acid / water solution to the flask and continuously shake the flask at low speed for 1 hour.
4. Dilute to volume with 1% acetic acid / water solution and mix well.
5. Filter an aliquot using a 0.45µm Millipore Millex PVDF filter. Discard the first 1-2 mL of filtrate before collection the filtrate for the further dilution.
6. Pipet 6 mL of the filtrate into a 50-mL volumetric flask, dilute to volume with 1% acetic acid / water and mix well.

### Analysis of Phenylephrine

Inject standards (0.00025 mg/mL of phenylephrine HCl in 1% acetic acid / water) and samples onto a suitable HPLC system under conditions similar to those suggested below. Parameters may be modified to optimize chromatography Determine the amount of the degradation products of phenylephrine HCl using the peak areas of the sample solutions under test in comparison with the peak areas of the standard solution.

### HPLC Chromatographic Conditions

| | | | | |
|---|---|---|---|---|
| Column | Supelco Ascentis RP-Amide, 250 mm length x 4.6 mm ID, 5 µm particle size, 100 Angstrom pore size | | | |
| Mobile Phase | A: [100 mM Ammonium Formate Buffer pH 2.9:Acetonitrile (99:1)] | | | |
| | B: [100 mM Ammonium Formate Buffer pH 2.9:Acetonitrile (50:50)] | | | |
| Mobile Phase | | Linear Gradient Program | | |
| Program | Flow | Time (minutes) | A (% Volume) | B (% Volume) |
| | 1.0 | 0 | 100 | 0 |
| | 1.0 | 10 | 100 | 0 |
| | 1.0 | 13 | 91 | 9 |
| | 1.0 | 21 | 45 | 55 |
| | 1.0 | 38 | 25 | 75 |
| | 1.0 | 43 | 0 | 100 |
| | 1.0 | 44 | 100 | 0 |
| | 1.0 | 50 | 100 | 0 |
| Detector | UV, 270 nm | | | |
| Injection Volume | 100 µL | | | |
| Column Temperature | Ambient | | | |
| Suggested Run Time | 50 minutes | | | |
| Approx. Retention Time | 4,6-ISOQUIN | 5.4 min | | |
| | 4,8-ISOQUIN | 6.7 min | | |
| | PHE-ONE | 9.4 min | | |
| | 3HOBA | 25.7 min | | |

### Example 2: Preparation of Coated Phenylephrine Resinate Extended Release Particles

Particles that contain phenylephrine and a cationic exchange resin were prepared and further coated with a semipermeable membrane. The ratio of the amounts of the coating ingredients, which can be varied to some extent, can be, e.g., cellulose acetate:hydroxypropylcellulose 2:1, 3:1, 4:1 or 5:1. The coating level, which can be varied to some extent, can be, e.g., 50%, 45%, 40%, 35%, 30%, 25% or 20% by weight of the coated particle. Most of the particles in the starting cation exchange resin had particle sizes between about 74 µm and about 177 µm (microns).

The phenylephrine resinate particles, which provide release of phenylephrine over an extended period of time, have proven to be stable at 25°C/60%RH for 24 months and at 40°C/75%RH for 3 months. Many granulated formulations of phenylephrine are not stable over time and undergo significant degradation.

A batch of 3.846 kg of coated phenylephrine resinate particles was prepared according to the formula in Table 3. The quantitative formula and batch formula are represented in Table 3 and Table 4, respectively.

**Table 3: Coated Phenylephrine Resinate Quantitative Formula**

| Component | Formula A¹ mg/Unit | Formula B¹ mg/Unit | Weight % (w/w) |
|---|---|---|---|
| Phenylephrine HCL USP | 20.00 | 15.00 | 19.50² |
| Sodium Polystyrene Sulfonate USP (most of the particles have a particle size of about 74 µm to about 177 µm) | 38.32 | 28.76 | 45.50 |
| Cellulose Acetate NF | 22.10 | 16.59 | 26.25 |
| Hydroxypropyl Cellulose NF | 7.37 | 5.53 | 8.75 |
| Acetone NF³ | -- | -- | -- |
| Purified Water USP³ | -- | -- | -- |

| | | | |
|---|---|---|---|
| 1: Unit doses of particles containing 20 mg (A) and 15 mg (B) phenylephrine HCl are approximately 84.2 mg and 63.2 mg respectively. Actual weight is dependent on the assayed amount of phenylephrine HCl in the particles. 2: Quantity represents the free base (1 mg of phenylephrine HCl is equivalent to 0.821 mg of phenylephrine free base). 3: Acetone and purified water are removed during processing. | | | |

**Table 4: Coated Phenylephrine Resinate Batch Formula**

| Component | Weight (kg)/Batch | Weight % (w/w) |
|---|---|---|
| Phenylephrine free base¹ | 0.750 | 19.5 |
| Sodium Polystyrene Sulfonate USP (particle size of about 74 µm to about 177 µm) | 1.750 | 45.5 |
| Cellulose Acetate NF | 1.0095 | 26.25 |
| Hydroxypropyl Cellulose NF | 0.3365 | 8.75 |
| Acetone NF³ | -- | -- |
| Purified Water USP³ | -- | -- |
| Total | 3.846 | 100.0 |

| | | |
|---|---|---|
| 1: One gram of phenylephrine hydrochloride is equivalent to 0.821 grams of phenylephrine free base. 2: Acetone and purified water are removed during processing. | | |

The coated phenylephrine resinate particles were produced using the following processing steps:

### Screening:

1. Sodium polystyrene sulfonate USP having desired particle size was passed through a 170 mesh screen and the fraction remaining on the screen was collected.

### Washing:

2. Sodium polystyrene sulfonate USP from Step 1 was dispersed in purified water and mixed.
3. While mixing, a portion of the slurry from Step 2 was filtered and washed with purified water USP. Filtration was continued until most of the water was removed.
4. The resin was transferred into a container.
5. Steps 3 and 4 were repeated until all of the slurry was removed.

### Drug Loading:

6. Purified water USP was added into a suitably sized stainless steel container.
7. While mixing, phenylephrine HCL was added to the container and mixed until dissolved.
8. The washed resin from Step 5 was added with continuous mixing and mixed into the slurry.
9. While mixing, a portion of the slurry from Step 8 was withdrawn and washed with purified water USP. Filtration was continued until most of the water was removed.
10. The washed filtered phenylephrine resinate from Step 9 was transferred into a container.
11. Steps 9 and 10 were repeated until all of the slurry was filtered.

### Drying:

12. The phenylephrine resinate was dried.

### Preparation of the coating solution:

13. Purified water USP and acetone NF were added to an appropriately sized stainless steel container.
14. Hydroxypropylcellulose NF was slowly added to the container and mixed until dissolved. Cellulose acetate NF was slowly added and mixed until dissolved.
15. Acetone NF was added until the solution was at the desired weight.

### Coating:

16. The phenylephrine resinate from Step 12 was coated with coating solution from Step 15 in appropriate fluid bed coating equipment fitted with a Wurster column.
17. The coated phenylephrine resinate was discharged into a container.

### Drying:

18. The coated phenylephrine resinate was dried.

### Screening:

19. The dried coated phenylephrine resinate was screened through a U.S. standard #40 mesh screen and the fraction passing through the screen was collected.

### Dissolution Analysis

The coated phenylephrine resinate particles from Step 19 were analyzed for dissolution from 0 to 14 hours using the apparatus described in United States Pharmacopeia <General Chapter <711> Dissolution>, Apparatus II, rotating paddles, utilizing UV detection at 274 nm. The dissolution media was 750 mL of 0.1N HCl for the first hour and was 1000 mL of a 0.05 M sodium phosphate buffer, pH 6.8, the second to the fourteenth hour. The temperature was 37°C and the rotation speed was 50 rpm. The dissolution showed that the percent of phenylephrine released versus a standard prepared at 100% of the amount of phenylephrine in the formulation was less than or equal to 50% in 1 hour, greater than or equal to 30% in 3 hours and greater than or equal to 50% in 8 hours. The method employed is below and the results are shown in Table 5 below.

### Dissolution method USP Apparatus 2 (paddles), 50 rpm

1. Verify that the dissolution media temperature has reached the target value.
2. Add sample (onto the surface of the medium solution) to each vessel containing 750 mL of 0.1 N hydrochloric acid and start the dissolution test with the paddle speed at 50 rpm. After 1 hour of operation in 0.1 N hydrochloric acid, pull the 1 hour sample, and proceed immediately to the buffer stage by adding 250 mL of 0.20 M tribasic sodium phosphate. The pH of the media should be 6.8 ± 0.05.
3. Pull 10 mL of dissolution sample solutions from each vessel after 1 hour, 3 hours, 6 hours (optional), and 8 hours. Filter the sample solutions through Varian Full Flow Filters (10 µm).
4. The amount of phenylephrine dissolved can be determined from UV absorbance in comparison with that of the standard solution at the wavelength of 274 nm. The amount of phenylephrine dissolved can also be determined using the phenylephrine assay method.
5. Correct the amount dissolved at 3, 6, and 8 hours by adding the amount pulled at the earlier time points. Use DISSL Program (or equivalent) or manually correct for intermediate sampling.

**Table 5**

| Time (hours) | % Dissolution |
|---|---|
| 1 | 20%-40% |
| 2 | 40%-60% |
| 3 | 50%-70% |
| 4 | 60%-80% |
| 6 | 75%-95% |
| 8 | 80%-100% |
| 10 | 90%-100% |
| 12 | 90%-100% |
| 14 | 90%-100% |

### Example 3: Particle Size Distribution Analysis

Several lots of resin and resin based particles were analyzed for particle size distribution. The samples included (1) Amberlite™ IRP69 resin, commercially available from The DOW Chemical Company, (2) unloaded resin having selected particle sizes (as prepared by Process A or Process B, respectively), and (3) loaded resinate particles (i.e., containing phenylephrine). The particle size distribution was analyzed using approximately 75 grams per sample in an FMC Syntron Sieve analyzer (FMC Technologies, Houston, TX), with settings at 90 volts for 11 minutes. The sieves were treated with a light dusting of magnesium stearate to prevent sticking during operation. The results are shown in Tables 6 and 7.

Particle size distribution can be analyzed on a smaller scale, using, e.g., an ATM L3P Sonic Sifter (Advantech Manufacturing, New Berlin, WI), which operates by using sonic pulses combined with mechanical agitation, to provide effective separation of particles.

**Table 6: Particle Size Analysis of Unloaded Resin with "As is" and Selection Process A & B**

| Mesh | Size | AL IRP69 Lot 1¹ | AL IRP69 Lot 2¹ | Lot 1 Process A² | Lot 2 Process A² | Lot 3 Process B³ | Lot 4 Process B³ |
|---|---|---|---|---|---|---|---|
| 80 | 177 µm | 0.1% | 0.2% | 0.1% | 0.1% | 0.2% | 0.0% |
| 100 | 149 µm | 0.2% | 0.3% | 0.4% | 0.4% | 2.0% | 0.2% |
| 120 | 125 µm | 0.8% | 0.9% | 3.4% | 3.4% | 19.8% | 14.5% |
| 140 | 105 µm | 1.8% | 1.7% | 13.3% | 15.1% | 35.0% | 34.1% |
| 200 | 74 µm | 23.2% | 18.2% | 80.9% | 79.4% | 41.7% | 50.4% |
| 325 | 44 µm | 38.1% | 37.4% | 1.7% | 1.5% | 1.3% | 0.8% |
| PAN | 0 µm | 35.8% | 41.4% | 0.1% | 0.0% | 0.0% | 0.0% |
| | >149µm | 0.4% | 0.5% | 0.6% | 0.5% | 2.2% | 0.2% |
| | <74µm | 73.9% | 78.7% | 1.8% | 1.5% | 1.3% | 0.8% |
| | >44µm | 64.2% | 58.6% | 99.9% | 100.0% | 100.0% | 100.0% |
| | 74-177 µm | 26.0% | 21.1% | 98.1% | 98.4% | 98.6% | 99.1% |
| | 74-125 µm | 24.9% | 19.9% | 94.2% | 94.6% | 76.8% | 84.5% |
| | D₁₀(µm)⁴ | 7.7 | 7.0 | 76.7 | 76.8 | 79.6 | 78.9 |
| | D₅₀(µm)⁴ | 53.4 | 49.6 | 91.1 | 91.6 | 108.7 | 104.1 |
| | D₉₀(µm)⁴ | 94.4 | 91.9 | 115.5 | 116.6 | 139.0 | 132.4 |
| | Mean (µm) | 54.8 | 51.5 | 94.4 | 94.6 | 108.8 | 105.0 |

| | | | | | | | |
|---|---|---|---|---|---|---|---|
| 1. Amberlite™ IRP69 "As is" commercially available 2. Amberlite™ IRP69 after selecting particle size "Process A" 3. Amberlite™ IRP69 after selecting particle size "Process B" 4. D₁₀, D₅₀ and D₉₀ determined using GRADISTAT, Blott, S.J. and Pye, K. (2001) GRADISTAT: a grain size distribution and statistics package for the analysis of unconsolidated sediments. Earth Surface Processes and Landforms 26, 1237-1248. | | | | | | | |

**Table 7: Particle Size Analysis of Loaded Resin with Selection Process A & B**

| Mesh | Size | Lot 1 Proc ess A¹ | Lot 2 Process A¹ | Lot 3 Process B² | Lot 4.1 Process B² | Lot 4.2 Process B² | Lot 4.3 Process B² | Lot 4.4 Process B² |
|---|---|---|---|---|---|---|---|---|
| 80 | 177 µm | 0.2% | 0.2% | 0.7% | 0.1% | 0.0% | 0.1% | 0.1% |
| 100 | 149 µm | 1.1% | 1.2% | 6.0% | 1.0% | 1.0% | 1.1% | 1.5% |
| 120 | 125 µm | 7.5% | 8.1% | 34.4% | 33.8% | 33.1% | 34.9% | 36.6% |
| 140 | 105 µm | 24.5 % | 32.3% | 36.5% | 36.7% | 38.1% | 36.1% | 33.2% |
| 200 | 74 µm | 65.8 % | 53.1% | 22.1% | 27.7% | 27.2% | 26.8% | 25.4% |
| 325 | 44 µm | 0.9% | 4.2% | 0.2% | 0.8% | 0.6% | 1.0% | 2.6% |
| PAN | 0 µm | 0.0% | 1.0% | 0.0% | 0.0% | 0.0% | 0.1% | 0.7% |
| | >149µm | 1.3% | 1.4% | 6.7% | 1.1% | 1.0% | 1.1% | 1.5% |
| | <74µm | 0.9% | 5.1% | 0.2% | 0.8% | 0.7% | 1.1% | 3.3% |
| | >44µm | 100. 0% | 99.0% | 100.0% | 100.0% | 100.0% | 99.9% | 99.3% |
| | 74-177 µm | 98.9 % | 94.6% | 99.1% | 99.2% | 99.3% | 98.8% | 96.7% |
| | 74-125 µm | 90.3 % | 85.3% | 58.6% | 64.3% | 65.3% | 62.9% | 58.6% |
| | D₁₀(µm)³ | 77.7 | 76.4 | 86.4 | 83.2 | 83.5 | 83.1 | 81.2 |
| | D₅₀(µm)³ | 96.1 | 99.5 | 119.8 | 116.3 | 116.2 | 116.8 | 117.4 |
| | D₉₀(µm)³ | 123. 9 | 124.7 | 146.5 | 142.3 | 142.1 | 142.5 | 143.1 |
| | Mean (µm) | 99.8 | 99.9 | 120.2 | 115.3 | 115.3 | 115.5 | 114.7 |

| | | | | | | | | |
|---|---|---|---|---|---|---|---|---|
| 1. Amberlite™ IRP69 after selecting particle size "Process A" 2. Amberlite™ IRP69 after selecting particle size "Process B" 3. D₁₀, D₅₀ and D₉₀ determined using GRADISTAT, Blott, S.J. and Pye, K. (2001) GRADISTAT: a grain size distribution and statistics package for the analysis of unconsolidated sediments. Earth Surface Processes and Landforms 26, 1237-1248. | | | | | | | | |

The impact of the drug-resin ratio on the efficiency of drug loading was observed. The results are shown in Tables 8 and 9 below.

**Table 8: Impact of drug-resin ratio on drug loading process for representative drug loading batches**

| **Lot** | **Size Range (µm)** | **Resin (kg)** | **Mixing Time (hr)** | **Drug-resin Ratio (Slurry)** | **Phenylephrine free base (%w/w) in Resinate** | **Loading Efficiency (%) of Resinate** | **Drug-resin Ratio (Resinate)** |
|---|---|---|---|---|---|---|---|
| Lot 4 Process B (Table 6) | 75-150 | 10.000 | 4.5 | 0.90:1 | 33.46 | 67.8% | 0.50:1 |
| Lot 3 Process B (Table 6) | 75-150 | 8.798 | 3.0 | 0.75:1 | 31.19 | 73.8% | 0.45:1 |
| Lot 4 Process B (Table 6) | 75-150 | 10.000 | 3.0 | 0.55:1 | 27.39 | 83.8% | 0.38:1 |
| Lot 4 Process B (Table 6) | 75-150 | 10.000 | 3.0 | 0.55:1 | 26.99 | 82.1% | 0.37:1 |
| Lot 4 Process B (Table 6) | 75-150 | 10.000 | 3.0 | 0.55:1 | 28.03 | 86.5% | 0.39:1 |
| Lot 4 Process B (Table 6) | 75-150 | 9.000 | 3.0 | 0.55:1 | 27.62 | 84.8% | 0.38:1 |
| Lot 4 Process B (Table 6) | 75-150 | 9.000 | 3.0 | 0.55:1 | 27.74 | 85.3% | 0.38:1 |
| Larger particle size | 212-420 | 0.500 | 3.0 | 0.69:1 | 30.36 | 76.7% | 0.44:1 |
| Larger particle size | 212-420 | 4.551 | 3.0 | 0.55:1 | 27.80 | 85.5% | 0.39:1 |
| Larger particle size | 212-420 | 4.750 | 3.0 | 0.55:1 | 27.97 | 86.2% | 0.39:1 |

**Table 9: Summary of drug-resin ratio impact on drug loading process**

| **Size Range (µm)** | **Mixing Time (hr)** | **Drug-resin Ratio (Slurry)¹** | **Phenylephrine free base (%) in Resinate** | **Loading Efficiency (%) of Resinate** | **Drug-resin Ratio (Resinate)** | **Comment(s)** |
|---|---|---|---|---|---|---|
| 75-150 | 3.0 | 0.55:1 | 27.55 | 84.5% | 0.38:1 | Average of 5 lots |
| | 3.0 | 0.75:1 | 31.19 | 73.8% | 0.45:1 | |
| | 4.5 | 0.90:1 | 33.46 | 67.8% | 0.50:1 | |
| 212-420 | 3.0 | 0.55:1 | 27.89 | 85.8% | 0.39:1 | Average of 2 lots |
| | 3.0 | 0.69:1 | 30.36 | 76.7% | 0.44:1 | |

| | | | | | | |
|---|---|---|---|---|---|---|
| 1. Based on phenylephrine HCl in slurry. 2. Based on free base of phenylephrine. | | | | | | |

### Phenylephrine Assay Method - Measurements for Tables 8 and 9

### Sample preparation

1. Accurately weigh suitable amount of coated phenylephrine resinate sample (containing equivalent of 25 mg of phenylephrine HCl) and transfer the weighed sample into a 500-mL volumetric flask.
2. Add 400 mL of diluent (1N HCl); shake the flask on a platform shaker at low speed for not less than 2 hours.
3. To ensure that particles do not collect above the solvent level, periodically rinse particulates into solution with diluent.
4. Dilute to volume with diluent and mix well.
5. Filter an aliquot using a 0.45 µm Millipore Millex PVDF syringe filter, or equivalent.
   Discard approximately the first 5 mL of filtrate before collection of the remainder in a HPLC vial for analysis.

### Analysis of Phenylephrine

Inject standards (0.05 mg/mL of phenylephrine HCl in 1 N HCl) and samples onto a suitable HPLC system under conditions similar to those suggested below. Parameters may be modified to optimize chromatography. Analytical results are valid if system suitability specifications are met.

### HPLC Chromatographic Conditions

| Column | Phenomenex Luna SCX, 100 mm length x 4.6 mm ID, 5 µm particle size, 100 angstrom pore size |
|---|---|
| Mobile Phase | 25 mM sodium acetate trihydrate buffer (pH 4.6):Acetonitrile (65:35, v/v) |
| Mobile Phase Program | Isocratic |
| Detector | UV, 214 nm |
| Flow Rate | 2.0 mL/min |
| Injection Volume | 100 µL |
| Column Temperature | Ambient |
| Suggested Run Time | 7 minutes |
| Approx. Retention Time | PHE 5 min |

### Example 4 - Dissolution Analysis of PK Study Material

The coated phenylephrine resinate particles employed in the first PK study, the second PK study and the PD study of Example 5 were analyzed for dissolution from zero to 8 hours using the method described in Example 2. The results are shown in Table 10A below.

**Table 10A: Dissolution Analysis (50 rpm)**

| Time point | Coated Phenylephrine Resinate Sample from PK Study 1 and PK Study 2 35% Coating Level 3:1 Cellulose Acetate NF Hydroxypropyl Cellulose NF (n = 6) % Released | Coated Phenylephrine Resinate Sample from PD Study 40% Coating Level 3:1 Cellulose Acetate NF Hydroxypropyl Cellulose NF (n=6) % Released |
|---|---|---|
| 1 Hour | 20-40% | 20-40% |
| 3 Hours | 50-70% | 50-70% |
| 6 Hours | 75-95% | 75-95% |
| 8 Hours | 80-100% | 80-100% |

The coated phenylephrine resinate particles employed in the first PK study, the second PK study and the PD study of Example 5 were also analyzed for dissolution from zero to 8 hours using the method described below. The results are shown in Table 10B below.

### Dissolution method USP Apparatus 2 (paddles), 75 rpm

1. Verify that the dissolution media temperature has reached the target value.
2. Add sample (directly into the medium solution using a suitable tube) to each vessel containing 750 mL of 0.1 N hydrochloric acid and start the dissolution test with the paddle speed at 75 rpm. After 1 hour of operation in 0.1 N hydrochloric acid, pull the 1 hour sample, and proceed immediately to the buffer stage by adding 250 mL of 0.20 M tribasic sodium phosphate. The pH of the media should be 6.8 ± 0.05.
3. Pull 10 mL of dissolution sample solutions from each vessel after 1 hour, 3 hours, 6 hours (optional), and 8 hours. Filter the sample solutions through Varian Full Flow Filters (10 µm).
4. Determine the amount of phenylephrine dissolved from UV absorbance in comparison with that of the standard solution at the wavelength of 274 nm.

The amount of phenylephrine dissolved can also be determined using the phenylephrine assay method.
5. Correct the amount dissolved at 3, 6, and 8 hours by adding the amount pulled at the earlier time points. Use DISSL Program (or equivalent) or manually correct for intermediate sampling.

**Table 10B: Dissolution Analysis (75 rpm)**

| Time point | Coated Phenylephrine Resinate Sample from PK Study 1 and PK Study 2 35% Coating Level 3:1 Cellulose Acetate NF Hydroxypropyl Cellulose NF (n = 6) % Released | Coated Phenylephrine Resinate Sample from PD Study 40% Coating Level 3:1 Cellulose Acetate NF Hydroxypropyl Cellulose NF (n=6) % Released |
|---|---|---|
| 1 Hour | 20-40% | 20-40% |
| 3 Hours | 70-90% | 65-85% |
| 6 Hours | 80-100% | 80-100% |
| 8 Hours | 90-100% | 90-100% |

### Stability Analysis

The coated phenylephrine resinate particles employed in the first PK study and the second PK study of Example 5 were analyzed for stability after storage at 1 month at 25°C and 60% relative humidity and at 1 month at 40°C and 75% relative humidity. For all samples, the levels of 3-hydroxybenzaldehyde were less than or equal to 0.5%; the levels of phenylephrine 4,6 isomer (N-Methyl-4, 6-Dihydroxy-1,2,3,4-tetrahydroxyisoquinolone HCL) and phenylephrine 4,8 isomer (N-Methyl-4, 8-Dihydroxy-1,2,3,4-tetrahydroxyisoquinolone HCL) were less than or equal to 2.0%. The total degradation product quantitated as related to phenylephrine was less than or equal to 2.0% at 1 month in each environment.

### Degradation Products Method

### Sample Preparation for Degradation Products Method

1. Accurately weigh suitable amount of coated phenylephrine resinate sample (containing equivalent of 25 mg of phenylephrine HCl) and transfer the weighed sample into a 500-mL volumetric flask.
2. Add 400 mL of diluent (1N HCl); shake the flask on a platform shaker at low speed for not less than 2 hours.
3. To ensure that particles do not collect above the solvent level, periodically rinse particulates into solution with diluent.
4. Dilute to volume with diluent and mix well.
5. Filter an aliquot using a 0.45 µm Millipore Millex PVDF syringe filter, or equivalent. Discard approximately the first 5 mL of filtrate before collection of the remainder in a HPLC vial for analysis.

### Analysis of Phenylephrine for Degradation Products Method

Inject standards (0.00025 mg/mL of phenylephrine HCl in 1 N HCl) and samples onto a suitable HPLC system under conditions similar to those suggested below. Parameters may be modified to optimize chromatography. Analytical results are valid if system suitability specifications are met.

### HPLC Chromatographic Conditions

| | | | | |
|---|---|---|---|---|
| Column | Supelco Ascentis RP-Amide, 250 mm length x 4.6 mm ID, 5 µm particle size, 100 Angstrom pore size | | | |
| Mobile Phase | A: [100 mM Ammonium Formate Buffer pH 2.9:Acetonitrile (99:1)] | | | |
| | B: [100 mM Ammonium Formate Buffer pH 2.9:Acetonitrile (50:50)] | | | |
| Mobile Phase Program | | Linear Gradient Program | | |
| | Flow | Time (minutes) | A (% Volume) | B (% Volume) |
| | 1.0 | 0 | 100 | 0 |
| | 1.0 | 10 | 100 | 0 |
| | 1.0 | 13 | 91 | 9 |
| | 1.0 | 21 | 45 | 55 |
| | 1.0 | 38 | 25 | 75 |
| | 1.0 | 43 | 0 | 100 |
| | 1.0 | 44 | 100 | 0 |
| | 1.0 | 50 | 100 | 0 |
| Detector | UV, 270 nm | | | |
| Injection Volume | 100 µL | | | |
| Column Temperature | Ambient | | | |
| Suggested Run Time | 50 minutes | | | |
| Approx. Retention Time | 4,6-ISOQUIN | 5.4 min | | |
| | 4,8-ISOQUIN | 6.7 min | | |
| | PHE-ONE | 9.4 min | | |
| | 3HOBA | 25.7 min | | |

### Example 5: Clinical Studies

Two pharmacokinetic (PK) studies and a pharmacodynamic (PD) study were conducted.

### A. First PK Study

A pilot study was conducted on sixteen subjects to determine the pharmacokinetic profile, bioavailability and metabolism of the coated extended release phenylephrine particles from Example 1 and the coated extended release phenylephrine resinate particles from Example 2. The subjects were assigned to receive four treatments after an overnight fast. There was a seven day washout between the four periods. In both cases, coated particles equivalent to a 20 mg phenylephrine HCl dose were administered in applesauce to healthy subjects. In addition, a combination of the extended release phenylephrine resinate particles from Example 2 and a commercial immediate-release liquid was evaluated. In the combination treatment, coated phenylephrine resinate particles equal to 15 mg phenylephrine HCl were administered in applesauce, and 10 mL of liquid equal to 5 mg phenylephrine HCl was administered by oral syringe.

The coated extended release phenylephrine particles from Example 1 and the coated extended release phenylephrine resinate particles from Example 2 were compared to McNeil-PPC, Inc.'s Non-Drowsy Children's Sudafed PE® Nasal Decongestant liquid (phenylephrine HCl 2.5mg/5mL). Table 11 summarizes the treatments in the first PK study.

**Table 11**

| Treatment Group | Total Dose Over 8 Hours |
|---|---|
| A (test) | coated ER phenylephrine resinate particles containing 20 mg phenylephrine |
| B (test) | coated ER phenylephrine HCl particles containing 20 mg phenylephrine |
| C (test) | coated ER phenylephrine resinate particles containing 15 mg phenylephrine and liquid IR phenylephrine HCl¹ containing 5 mg phenylephrine |
| D (reference) | liquid IR phenylephrine HCl containing 10 mg phenylephrine |

| | |
|---|---|
| ¹ The unit dose of approximately 84.2 mg coated ER phenylephrine resinate particles is equivalent to a 20 mg phenylephrine HCl dose. The unit dose of approximately 63.2 mg coated ER phenylephrine resinate particles is equivalent to a 15 mg phenylephrine HCl dose, and this latter unit dose was administered with 10 mL phenylephrine liquid 2.5 mg/5mL, for a total of dose equivalent to a 20 mg phenylephrine HCl. | |

The coated ER phenylephrine resinate particles and the ER phenylephrine HCl particles were administered orally after folding the measured amount into a 4 oz cup of applesauce just prior to dosing. These single doses were swallowed without chewing, and followed with 240 mL of water. The phenylephrine HCl liquid was administered orally using an oral syringe. To standardize the conditions for dosing the reference treatment, the first of two oral 10 mg doses of liquid were followed with a 4 oz cup of applesauce and 240 mL of water.

Serial blood samples were collected into K3-EDTA tubes at specific time points over 8 or 16 hours after dose.

### B. Second PK Study

A second pilot study was conducted: (i) to determine if 30 mg phenylephrine can attain similar maximum drug concentrations relative to two 10 mg doses of immediate-release phenylephrine given 4 hours apart; and (ii) to evaluate the ER PK profile and bioavailability of 20 mg phenylephrine and 1300 mg acetaminophen.

The second pilot study was conducted on twenty subjects to determine the pharmacokinetic profiles, bioavailability and metabolism of (1) a combination of (a) the coated extended release phenylephrine resinate particles from Example 2 equal to 15 mg phenylephrine HCl, (b) 10 mL phenylephrine liquid equal to 5 mg phenylephrine HCl and (c) 1300 mg extended release acetaminophen; (2) a combination of (a) the coated extended release phenylephrine resinate particles from Example 2 equal to 22.55 mg phenylephrine HCl and (b) phenylephrine liquid equal to 7.5 mg phenylephrine HCl; (3) a combination of (a) phenylephrine liquid equal to 20 mg phenylephrine HCl and (b) 1300 mg extended release acetaminophen; and (4) phenylephrine liquid equal to 20 mg phenylephrine HCl. Table 12 summarizes the treatments in the second PK study

**Table 12**

| Treatment Group | Total Dose Over 8 Hours |
|---|---|
| A (test) | coated ER phenylephrine resinate containing 15 mg phenylephrine and liquid IR phenylephrine HCl containing 5 mg phenylephrine and 1300 mg ER acetaminophen² |
| B (test) | coated ER phenylephrine resinate containing 22.5 mg phenylephrine and liquid IR phenylephrine HCl containing 7.5 mg phenylephrine |
| C (reference) | liquid IR phenylephrine HCl containing 20 mg phenylephrine and 1300 mg ER acetaminophen |
| D (reference) | liquid IR phenylephrine HCl containing 20 mg phenylephrine |

| | |
|---|---|
| ² The extended release acetaminophen tablet formulation was the same granulation formulation that is commercially available in Tylenol® Arthritis. | |

Serial blood samples were collected into K3-EDTA tubes at specified time points over 12 or 20 hours.

### Results

Results for the PK studies appear in Figures 1-11 and in Table 13 below.

**Table 13 - Comparison of Mean Parameters for First PK Study**

| | A | B | C | D |
|---|---|---|---|---|
| | (20 mg PE Resinate) | (20mg PE HCl) | (5 IR + 15mg ER Resinate) | (10mg IR Q4H x 2 doses) |
| AUC* (pg-hr/mL) | 1241.7 | 1162.6 | 1237.8 | 1162.5 |
| Cmax (pg/mL) | 208.4 | 219.4 | 230.8 | 458 |
| Tmax (hr) | 2.5 | 2.8 | 1.97 | Tmax 1 = 0.36 |
| | | | | Tmax 2 = 4.54 |

| | | | | |
|---|---|---|---|---|
| Note: Treatments A, B and C = AUC over 16 hours. Treatment D = AUC over 8 hours Figures are rounded off | | | | |

In sum, the results show that:
The ER-IR blend containing 20 mg phenylephrine had a Cmax that was 50% of the 10 mg IR dose and an AUCinf that was 15% more than two 10 mg IR doses (20 mg).
The ER-IR blend containing 30 mg phenylephrine had a Cmax that was 85% of the 10 mg IR dose and an AUCinf that was 61 % more than two 10 mg IR doses (20 mg).
The ER-IR Blend containing 20 mg phenylephrine and 1300 mg acetaminophen had a Cmax that was 80% of the 10 mg phenylephrine IR dose and an AUCinf that was 22% more than two 10 mg IR doses (20 mg).

The results demonstrate that the formulation of the present invention provides efficacy over an extended period of time.

These results also demonstrate that the formulation of the present invention is able to match the duration of extended release acetaminophen.

The results also demonstrate that phenylephrine exposure is increased and phenylephrine PK profile is improved relative to a 10 mg immediate release dose of phenylephrine when phenylephrine is combined with acetaminophen. This may be due to competition for gut wall metabolism leading to greater absorption of phenylephrine and no effect on acetaminophen; and extended release formulation providing greater absorption of phenylephrine due to avoidance of gut wall metabolism in lower GI tract.

### C. Pharmacodynamic Study

A randomized, double-blind, placebo-controlled study was conducted to determine the efficacy of phenylephrine and phenylephrine-acetaminophen extended release formulations in subjects with congestion and pain symptoms due to upper respiratory tract infections. A 30 mg ER dose, a 45 mg ER dose and a 30 mg ER dose co-administered with 1300 mg acetaminophen were assessed and compared to placebo. In each example, the coated ER phenylephrine resinate particles of the invention were employed. The 30 mg ER dose, the 45 mg ER dose and the 30 mg ER dose co-administered with 1300 mg acetaminophen each performed well against placebo in severity score for (1) stuffy/congested nose; (2) sinus pressure/tenderness; and (3) head congestion from 0 to 12 hours on Day 1.

The foregoing examples are not intended to limit the scope of the present invention, which may be set out in the claims. In particular, various equivalents and substitutions will be recognized by those skilled in the art in view of the foregoing disclosure and these are contemplated to be within the scope of the invention.

### References

Blackledge HM, O'Farrell J, Minton NA, et al. The effect of therapeutic doses of paracetamol on sulphur metabolism in man. Hum Exp Toxicol 1991 May; 10(3): 159-65.
Court MH, Duan SX, Von Moltke LL, et al. Interindividual variability in acetaminophen glucuronidation by human liver microsomes: Identification of relevant acetaminophen UDP-glucuronosyltransferase isoforms. J Pharmacol Exp Ther 2001; 299(3):998-1006.
Empey DW and Medder KT. Nasal Decongestants. Drugs 1981; 21:438-443.
Hengstmann JH, Goronzy J. Pharmacokinetics of 3H-phenylephrine in man. Eur J Clin Pharmacol 1982; 21:335-341.
Hoffman BB. Chapter 10: Catecholamines, Sympathomimetic Drugs, and Adrenergic Receptor Antagonists. In: Goodman & Gilman's The Pharmacologic Basis of Therapeutics - 10th Ed. Hardman JG and Limbird LE, eds. McGraw-Hill, Medical Publishing Division, USA, 2001.
Ibrahim KE, Midgley JM, Crowley IR, and Willaims CM. The mammalian metabolism of R-(-)-m-synephrine. J Pharm Pharmacol. 1983; 35:144-147.
Johnson DA, Hricik JG. The pharmacology of a-adrenergic decongestants. Pharmacother 1993; 13:110S-115S.
Koch-Weser J. Medical Intelligence: Drug Therapy. N Engl J Med 1976 Dec 2; 295(23):1297-1300.
Manyike PT, Kharasch ED, Kalhorn TF, et al. Contribution of CYP2E1 and CYP3A to acetaminophen reactive metabolite formation. Clin Pharmacol Ther 2000 Mar; 67(3):275-282.
Miners JO, Atwood J, Birkett DJ. Influence of sex and oral contraceptive steroids on paracetamol metabolism. Br J Clin Pharmacol 1983; 16:503-509.
Miners JO, Osborne NJ, Tonkin AL, et al. Perturbation of paracetamol urinary metabolic ratios by urine flow rate. Br J Clin Pharmacol 1992; 34:359-362.
Mitchell JR, Thorgeirsson SS, Potter WZ, et al. Acetaminophen-induced injury: Protective role of glutathione in man and rationale for therapy. Clin Pharmacol Ther 1974; 16:676-684.
Slattery JT, McRorie TI, Reynolds R, et al. Lack of effect of cimetidine on acetaminophen disposition in humans. Clin Pharmacol Ther 1989 Nov; 46(5):591-597.
Suzuki O. Matsumoto T. Oya M, Katsumata Y. Oxidation of synephrine by type A and type B monoamine oxidase. Experientia 1979; 35:1283-1284.

## Claims

1. An extended release particle, wherein said extended release particle comprises starch, phenylephrine, and ethyl acrylate and methyl methacrylate copolymer, wherein said starch, phenylephrine, and ethyl acrylate and methyl methacrylate copolymer are coated with at least two coating layers, wherein a first coating layer comprises ethylcellulose, acetyltributylcitrate and magnesium stearate, wherein a second coating layer comprises ethyl acrylate and methyl methacrylate copolymer and ethylcellulose aqueous dispersion.

2. The extended release particle of claim 1, wherein said extended release particle is stable at 25°C/60% relative humidity for 24 months and at 40°C/75% relative humidity for 3 months.

3. A composition containing the extended release particles of claim 1 or claim 2, wherein said composition comprises the ingredients set forth below:
| **Component** | **Weight/Unit Dose (mg)** | **Weight % (w/w)** |
|---|---|---|
| Phenylephrine HCl | 20.00 | 5.30 |
| Pregelatinized Modified Starch | 91.22 | 24.17 |
| Ethyl Acrylate and Methyl Methacrylate Copolymer Dispersion | 134.66 | 35.68 |
| Ethylcellulose | 45.33 | 12.01 |
| Acetyltributyl Citrate | 9.06 | 2.40 |
| Magnesium Stearate | 9.06 | 2.40 |
| Ethylcellulose, cetyl alcohol and sodium lauryl sulfate dispersion | 66.27 | 17.56 |
| Colloidal Silicon Dioxide | 1.77 | 0.47 |
| Purified Water | -- | -- |
| Acetone | -- | -- |
| Isopropyl Alcohol | -- | -- |
| TOTAL | | 100.00 |

## Patentansprüche

1. Partikel mit anhaltender Freisetzung, wobei das Partikel mit anhaltender Freisetzung Stärke, Phenylephrin und Ethylacrylat-Methylmethacrylat-Copolymer umfasst, wobei die Stärke, das Phenylephrin und das Ethylacrylat-Methylmethacrylat-Copolymer mit mindestens zwei Überzugsschichten beschichtet sind, wobei eine erste Überzugsschicht Ethylcellulose, Acetyltributylcitrat und Magnesiumstearat umfasst und wobei eine zweite Überzugsschicht Ethylacrylat-Methylmethacrylat-Copolymer und eine wässrige Ethylcellulosedispersion umfasst.

2. Partikel mit anhaltender Freisetzung nach Anspruch 1, wobei das Partikel mit anhaltender Freisetzung bei 25°C/60% relative Feuchtigkeit 24 Monate lang und bei 40°C/75% relative Feuchtigkeit 3 Monate lang stabil ist.

3. Zusammensetzung, enthaltend die Partikel mit anhaltender Freisetzung nach Anspruch 1 oder Anspruch 2, wobei die Zusammensetzung die unten aufgeführten Bestandteile umfasst:
| **Komponente** | **Gewicht/Einheitsdosis (mg)** | **Gew.-% (w/w)** |
|---|---|---|
| Phenylephrin HCl | 20,00 | 5,30 |
| vorgelatinisierte modifizierte Stärke | 91,22 | 24,17 |
| Ethylacrylat-Methylmethacrylat-Copolymerdispersion | 134,66 | 35,68 |
| Ethylcellulose | 45,33 | 12,01 |
| Acetyltributylcitrat | 9,06 | 2,40 |
| Magnesiumstearat | 9,06 | 2,40 |
| Dispersion von Ethylcellulose, Cetylalkohol und Natriumlaurylsulfat | 66,27 | 17,56 |
| kolloidales Siliciumdioxid | 1,77 | 0,47 |
| gereinigtes Wasser | -- | -- |
| Aceton | -- | -- |
| Isopropylalkohol | -- | -- |
| GESAMT | | 100,00 |

## Revendications

1. Particule à libération prolongée, où ladite particule à libération prolongée comprend de l'amidon, de la phényléphrine, et un copolymère d'acrylate d'éthyle et de méthacrylate de méthyle, où lesdits amidon, phényléphrine, et copolymère d'acrylate d'éthyle et de méthacrylate de méthyle, sont revêtus avec au moins deux couches de revêtement, où une première couche de revêtement comprend de l'éthylcellulose, du citrate d'acétyltributyle et du stéarate de magnésium, où une deuxième couche de revêtement comprend un copolymère d'acrylate d'éthyle et de méthacrylate de méthyle et une dispersion aqueuse d'éthylcellulose.

2. Particule à libération prolongée selon la revendication 1, où ladite particule à libération prolongée est stable à 25°C/60% d'humidité relative pendant 24 mois et à 40°C/75% d'humidité relative pendant 3 mois.

3. Composition contenant les particules à libération prolongée selon la revendication 1 ou la revendication 2, où ladite composition comprend les ingrédients exposés ci-après :
| **Composant** | **Poids/Dose Unitaire (mg)** | **% en Poids (p/p)** |
|---|---|---|
| Phényléphrine HCl | 20,00 | 5,30 |
| Amidon modifié prégélatinisé | 91,22 | 24, 17 |
| Dispersion de copolymère d'acrylate d'éthyle et de méthacrylate de méthyle | 134,66 | 35, 68 |
| Ethylcellulose | 45,33 | 12,01 |
| Citrate d'acétyltributyle | 9,06 | 2,40 |
| Stéarate de magnésium | 9,06 | 2,40 |
| Dispersion d'éthylcellulose, d'alcool cétylique et de laurylsulfate de sodium | 66,27 | 17,56 |
| Dioxyde de silicium colloïdal | 1,77 | 0,47 |
| Eau purifiée | - | - |
| Acétone | - | - |
| Alcool isopropylique | - | - |
| TOTAL | | 100,00 |
